Europäisches Patentamt

European Patent Office

Office européen des brevets

(19)

(11) Publication number: **0 128 703**
**B1**

(12) **EUROPEAN PATENT SPECIFICATION**

(45) Date of publication of patent specification: **07.01.88**

(51) Int. Cl.⁴: **A 61 M 5/14**

(21) Application number: **84303656.7**

(22) Date of filing: **31.05.84**

(54) **Non-contact controlled micropump.**

(30) Priority: **31.05.83 JP 97327/83**
**25.07.83 JP 115375/83**

(43) Date of publication of application:
**19.12.84 Bulletin 84/51**

(45) Publication of the grant of the patent:
**07.01.88 Bulletin 88/01**

(84) Designated Contracting States:
**CH DE FR GB LI NL**

(56) References cited:
**EP-A-0 048 423**
**WO-A-80/02377**
**FR-A-2 306 712**
**GB-A-1 604 576**
**GB-A-2 119 904**

**TRANSACTIONS OF THE AMERICAN SOCIETY**
**OF ARTIFICIAL INTERNAL ORGANS, vol. 24,**
**1978, pages 229-231; P.R. PERKINS et al.:**
**"Design and initial testing of a totally**
**implantable transcutaneously controllable**
**insulin delivery device"**
**IDEM**

(73) Proprietor: **Koichi, Sakurai**
**101 Aza-Motomachi Oaza-Terano Shinkawa-cho**
**Nishikasugai-gun Aichi-ken (JP)**

(73) Proprietor: **KUREHA KAGAKU KOGYO**
**KABUSHIKI KAISHA**
**9-11 Horidome-cho 1-chome Nihonbashi Chuo-ku**
**Tokyo (JP)**

(72) Inventor: **Kondo, Tatsuhei**
**3-57 Asaoka-cho Chikusa-ku**
**Nagoya-shi Aichi-ken (JP)**
Inventor: **Ito, Kaname**
**23-19 Aza-Higashikanakuso Saburi**
**Chita-shi Aichi-ken (JP)**
Inventor: **Ikeda, Shoichiro**
**3-2-334 Fujimaki-cho Meito-ku**
**Nagoya-shi Aichi-ken (JP)**
Inventor: **Umeno, Masayoshi**
**2-43-2 Nishizato-cho Meito-ku**
**Nagoya-shi Aichi-ken (JP)**
Inventor: **Ichikawa, Kenji**
**8-2 Takayama Tokitsu-cho**
**Toki-shi Gifu-ken (JP)**

(74) Representative: **Senior, Alan Murray et al**
**J.A. KEMP & CO 14 South Square Gray's Inn**
**London WC1R 5EU (GB)**

## Description

This invention relates to a micropump, particularly one which can be implanted in a body and controlled from outside the body.

More particularly, the invention relates to a micropump to be disposed within a human body for continuously delivering small quantities of a pharmaceutical liquid into the human body. With the invention the delivery rate can be controlled by the action of a pharmaceutical liquid injection control device in response to external electric signals.

Generally, pharmaceutical liquids are injected by one day's dose at a time. This is often excessive and a major amount is rapidly drained off. As a result, the remaining liquid is insufficient over the subsequent period.

The aim of this invention is to provide a micropump for continually directly delivering a pharmaceutical liquid in small quantities to a human body.

Transactions of the American Society of Artificial Internal Organs, Volume 24, 1978, pages 229 to 231 "Design and initial testing of a totally implantable transcutaneously controllable insulin delivering device" discloses a micropump in accordance with the prior art portion of claim 1. This prior art device is arranged to supply liquid continuously and the magnet controllable valve is used to direct the flow selectively through flow paths of differing resistance. This does not give the same reliable dosing as can be achieved having an accurately controllable on/off valve and also requires a relatively bulky restrictive flow path which can be liable to blockage. The present invention is characterised as set out in Claim 1. In addition to the advantageous provision of the on/off control under the control of an external electro-magnet the present invention also provides for the propellant to be received within the bellows and the liquid to be received between the bellows and the casing.

The micropump of the invention can operate in response to external electric signals, the micropump storing the doses of some days. These doses can be injected at once into a reservoir situated in the human body.

The invention is useful in various fields, particularly, for medical use in which the concentration of pharmaceuticals or essential substances in a living body should be constantly maintained.

For example, commonly used pharmaceuticals can remain at a constant concentration in blood if they are introduced at a constant rate. Also, in order to ensure a constant concentration of glucose in the blood, insulin delivery doses must always be changed in accordance with the patient's moment-to-moment needs, which vary in accordance with the conditions of the living body.

Conventional insulin-infusion pumps, which are externally placed and designed percutaneously to inject insulin, have many problems including the risk of infection via the pierced site on the skin, and considerably restrict the patient's ordinary activities.

With the invention, the pump body can be implanted in the living body, while the control part is externally placed. This eliminates the need repeatedly to pierce the skin for injecting a pharmaceutical liquid and reduces the risk of infection. The external control part including a power supply can be small enough and light as compared with conventional pump bodies.

Preferably, the micropump includes a pressure regulating valve for regulating the pressure of the liquid pressured by the bellows prior to supply of such liquid to the first said valve.

A preferred embodiment of this invention will now be described by way of example with reference to the accompanying drawings, in which:—

Figure 1 is a sectional view along line I—I of Figure 2 showing a pump of this invention;

Figure 2 is a plan view of the pump of Figure 1;

Figure 3 is a sectional view along line III—III of Figure 1;

Figure 4 is a sectional view along line IV—IV of Figure 5 showing a pressure regulating valve used in the pump;

Figure 5 is a plan view of the valve of Figure 4;

Figure 6 is a sectional view along VI—VI of Figure 7 showing an example incorporating the pressure regulating valve of Figure 4; and

Figure 7 is a plan view.

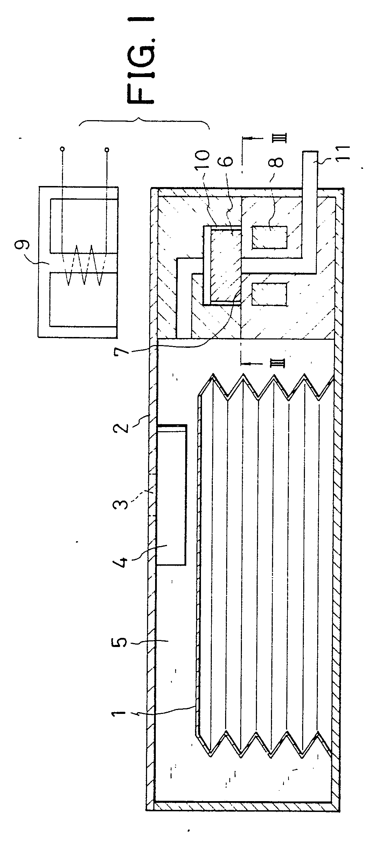

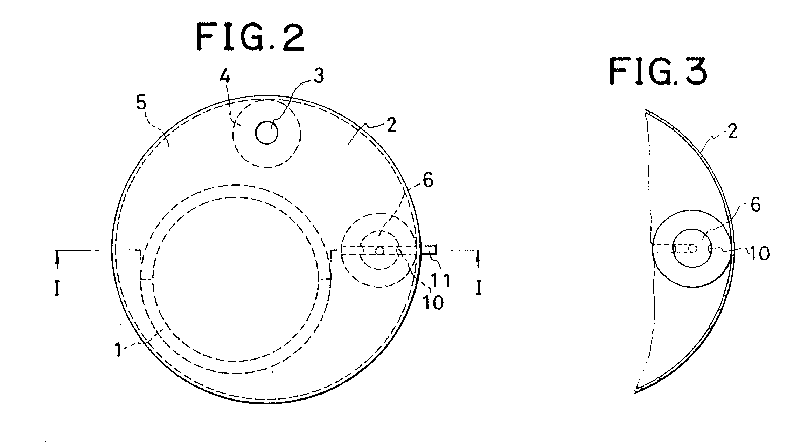

The apparatus shown in Figures 1 to 3 is designed to be implanted and includes a bellows 1 enclosing air or gas which in use is expanded by the increase of the air, gas or vaporized gas pressure therein as a result of the body temperature. A casing 2 houses a pharmaceutical liquid reservoir 5. A pharmaceutical liquid can be previously injected into the pharmaceutical liquid reservoir 5 via an inlet 3 through the casing 2 and a rubber packing 4 located against the inlet. Injection can be by means of an injector needle. As shown in Figure 2 the inlet 3 does not overlap the bellows 1 in the direction in which an injector needle would be inserted into the inlet 3 for the supply of liquid to the reservoir 5. When the bellows 1 is expanded, the pharmaceutical liquid tends to be expelled from chamber 5 and presses a permanent magnet valve 6 against a valve seat 7 thus closing an outlet 11 from the apparatus. The valve 6 is usually attracted magnetically towards a ferrite core 8 to prevent leakage of the pharmaceutical liquid. In order to open the valve 6, a force of magnetic attraction stronger than the the force applied by core 8 is applied by an external electromagnet 9 and lifts the valve 6 from the valve seat 7, whereon pharmaceutical liquid is released from the pharmaceutical liquid outlet 11 via a bypass path 10 round the valve 6. When the force of magnetic attraction produced by the electromagnet 9 is removed the valve 6 is attracted to the ferrite core 8 to close the valve seat and to stop the release of the pharmaceutical liquid. The dose released is controlled by the duration the electromagnet 9 is applied.

In this way, the pharmaceutical liquid infusion can be externally controlled as required and at any time, while there need be no difficulties due to, for instance, an exhausted battery because the power source for controlling the delivered quantities is located outside the body.

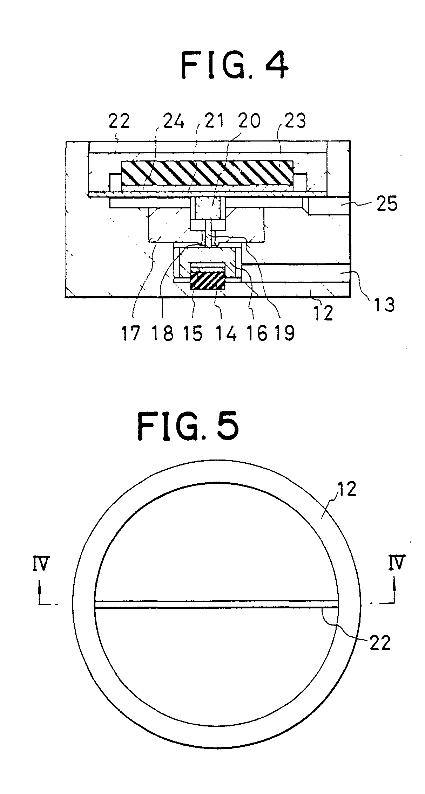

Figures 4 to 7 illustrate a pressure regulating valve for use in a delivery system as described above.

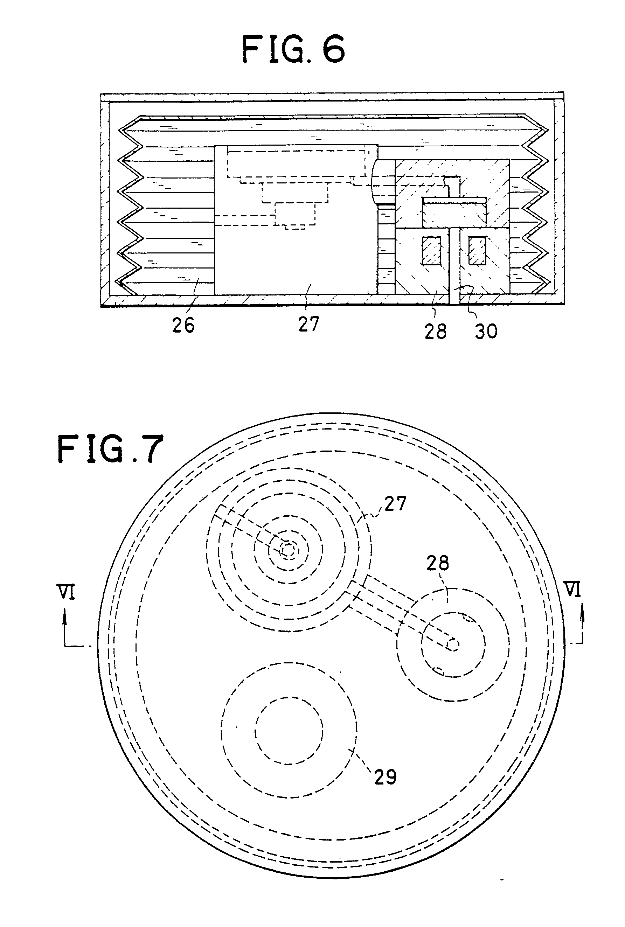

In order to inject only a very small quantity of pharmaceutical liquid it is necessary to have a small diameter of valve and to limit the pressure of the pharmaceutical liquid to be injected. For this purpose, it is possible to reduce the pressure to tolerable limits by means of a pressure regulating valve interposed between the permanent magnet valve and the pharmaceutical liquid reservoir, which is pressurised by the bellows. Figures 4 and 5 show a pressure regulating valve, of very small type, which can be used for this purpose, and Figures 6 and 7 show an example of application of such a pressure regulating valve to a micropump of the invention.

In Figure 4, a liquid of primary pressure enters through an inlet 13 of a body 12, and passes through a gap between the body 12 and a regulating valve 16, which is urged upward by the elastic force of a rubber member 14 and a pressure receiving plate 15. The liquid reaches a region of contact between the regulating valve 16 and a valve seat 18 of a sheet 17. The regulating valve 16 is pressed by a rod 20 connected to a diaphragm 21 and to a needle 19 placed at the center of the valve seat. The diaphragm 21 itself receives pressure from a pressure receiving plate 24 subject to the elastic force of a rubber plate 23 pressed by a cover 22.

The regulating valve 16 is urged upward by the elastic force $F_1$ of the rubber plate 14 and a force $AP_1$, where $F_1$ is a small force for firmly contacting the regulating valve 16 with the valve 18, $P_1$ is the primary pressure and A is the area of the valve seat 18. In practice the diaphragm 21 is at an initial position where the elastic force of the rubber plate 23 is adjusted so that the force thrusting the needle 19 downward under the pressure exerted on the diaphragm 21 is equal to the upward force of the regulating valve. When the elastic force of the rubber plate 23 is slightly increased, the diaphragm 21 is moved below the initial position and the needle 19 is urged on the regulating valve 16, thus causing the liquid at primary pressure to flow out from an outlet 25 via a gap around the needle 19 and the rod 20. When the outlet 25 is closed, the secondary pressure in the outlet is increased to move the diaphragm 21 up to its initial position. Accordingly, if the elastic force of the rubber plate 23 is adjusted in such a manner that the secondary pressure is equal to a desired pressure $P_2$, an expected constant pressure is obtained when the outlet is closed. When the outlet 25 is slightly opened, the liquid flows out and the secondary pressure $P_2$ is decreased. Thus, the diaphragm 21 is moved downward to allow the needle to open the regulating valve so that the liquid is delivered on the secondary pressure side. The secondary pressure is now increased and the diaphragm 21 restores the initial position, thereby closing the valve seat 18 to stop the liquid inflow. In this stage, the secondary pressure comes to an expected value, since the rubber plate 23 is arranged to ensure $P_2$. Accordingly, the secondary pressure is unchanged whether the inflow is nothing or at any rate.

Further, the secondary pressure is not changed even if the primary pressure is changed. The force applied to the diaphragm 21 is given by $(B-A)P_2$, where B is the area of the diaphragm 21. When the diaphragm 21 is at the initial position and all the forces are balanced,

$$(B-A)P_2=F_1+AP_1 \therefore P_2=\frac{F_1}{B-A}+\frac{A}{B-A}P_1$$

As $F_1$, B and A are constants, differentiation of the pressure P gives

$$P_2=\frac{A}{B-A}P_1$$

If the valve seat has a tenth of the diameter of the diaphragm, A is a hundredth of B and the variation of $P_2$ is only 1/99 of the variation of $P_1$. Accordingly, the pressure regulating valve as described above has the advantage that the secondary pressure variation can be ignored even if the primary pressure is changed.

Figures 6 and 7 show an example incorporating such a pressure regulating valve 27, as described above, as well as a pharmaceutical liquid reservoir 26, a valve 28, a pharmaceutical liquid inlet 29 and pharmaceutical liquid outlet 30.

## Claims

1. A micropump for implantation within a living body for delivering small quantities of a liquid, said micropump comprising a casing (2) divided by a bellows (1) into a reservoir (5) for receiving the liquid and a chamber for receiving a propellant, an inlet (3) for supplying liquid to the interior of the casing, an outlet (11) for releasing liquid from the casing and magnet controlled valve means (6, 7) for controlling the flow rate of the liquid from the outlet (11) to a predetermined value, characterized in that the reservoir is provided exteriorly of the bellows (1) in the casing (2) with the propellant receiving chamber being defined by the interior of the bellows, and in that the flow control valve is an on/off valve (6, 7) normally biased into a closed condition and operable by an external electromagnet (9).

2. A micropump according to Claim 1, characterized in that the liquid inlet (3) is so disposed that it does not overlap the bellows (1) in the direction in which an injector needle is insertable into the inlet for supplying the liquid thereto.

3. A micropump according to Claim 1 or 2, characterised in that flow regulating means (12, 14, 16, 18, 19, 21, 23, 24) are provided in the casing to control the rate of flow of liquid during opening of the flow control valve (6, 7).

4. A micropump according to Claim 3, characterised in that the flow regulating means is connected between the reservoir (5) and the flow control valve (6, 7) and comprises a valve member (16) urged towards a valve seat (18) by an elastic-force generating means (14) and urged away from the valve seat (18) to allow a passage of the liquid past the valve member (16) in response to a pressure exerted on a diaphragm (24) on the downstream side thereby to maintain a pressure of the liquid supplied to the flow control valve (6, 7) to a predetermined value.

5. A micropump according to Claim 4, characterised in that the valve seat (18) is formed in a cylindrical body having a central axial bore to direct the liquid towards the diaphragm (24) and an annular flat end surface facing the valve member (16), the annular flat surface being engageable in liquid-tight fashion with a flat surface of the valve member (16) which is parallel with said annular flat surface.

## Patentansprüche

1. Mikropumpe zur Implantation in einen lebenden Körper für die Abgabe von kleinen Flüssigkeitsmengen, wobei die Mikropumpe ein Gehäuse (2) aufweist, das durch einen Faltenbalg (1) in ein Reservoir (5) für die Aufnahme der Flüssigkeit und in eine Kammer für die Aufnahme eines Triebmittels unterteilt ist, mit einem Einlaß (3) für die Zufuhr der Flüssigkeit zu dem Inneren des Gehäuses, mit einem Auslaß (11) zum Abgeben der Flüssigkeit aus dem Gehäuse und mit einer magnetgesteuerten Ventileinrichtung (6, 7) zur Steuerung der Strömungsgeschwindigkeit der Flüssigkeit von dem Auslaß (11) auf einen vorgegebenen Wert, dadurch gekennzeichnet, daß das Reservoir außerhalb des Faltenbalgs (1) in dem Gehäuse (2) untergebracht ist, wobei die das Triebmittel aufnehmende Kammer von dem Inneren des Faltenbalgs gebildet wird, und daß das Strömungssteuerventil ein Ein/Aus Ventil (6, 7) ist, das normalerweise geschlossen ist und von einem externen Elektromagneten (9) betätigt wird.

2. Mikropumpe nach Anspruch 1, dadurch gekennzeichnet, daß der Flüssigkeitseinlaß (3) derart angeordnet ist, daß er den Faltenbalg (1) in der Richtung nicht überlappt, in der eine Injektornadel in den Einlaß einsetzbar ist, um die Flüssigkeit dorthinein zuzuführen.

3. Mikropumpe nach Anspruch 1 oder 2, dadurch gekennzeichnet, daß die Strömungsregelungsmittel (12, 14, 16, 18, 19, 21, 23, 24) in dem Gehäuse untergebracht sind, um die Strömungsgeschwindigkeit der Flüssigkeit während des Öffnens des Strömungssteuerventils (6, 7) zu steuern.

4. Mikropumpe nach Anspruch 3, dadurch gekennzeichnet, daß das Strömungsregulierungsmittel zwischen das Reservoir (5) und das Strömungssteuerventil (6, 7) geschaltet ist und ein Ventilelement (16) aufweist, das von einem eine Elastikkraft erzeugenden Mittel (14) auf einen Ventilsitz (18) gedrückt wird und von dem Ventilsitz (18) weggedrückt wird, um den Durchtritt der Flüssigkeit an dem Ventilelement (16) vorbei in Abhängigkeit von einem auf eine Membran (24) ausgeübten Druck an der stromabwärtigen Seite zu gestatten, damit der Druck der Flüssigkeit, die dem Strömungssteuerventil (6, 7) zugeführt wird, auf einem vorgegebenen Wert gehalten wird.

5. Mikropumpe nach Anspruch 4, dadurch gekennzeichnet, daß der Ventilsitz (18) in einem zylindrischen Körper gebildet ist, der eine zentrale Axialbohrung aufweist, um die Flüssigkeit zu der Membran (24) zu leiten und der eine dem Ventilelement (16) zugewandte ringförmige, ebene Endfläche aufweist, auf die sich eine ebene Fläche des Ventilelements (16), die parallel zu der ringförmigen ebenen Fläche verläuft, flüssigkeitsdicht auflegen kann.

## Revendications

1. Une micro-pompe pour implantation dans un corps human vivant pour le débit de petites quantités de liquide, la dite micro-pompe comprenant: un corps de pompe (2) divisé par un soufflet (1) dans un réservoir (5) pour la réception du liquide et par une chambre pour la réception d'un propulseur; une admission (3) pour la fourniture de liquide à l'intérieur du corps de pompe; un échappement (11) pour la sortie du liquide hors du corps de pompe; et un moyen de vanne à commande magnétique (6, 7) pour la commande du régime de débit du liquide à partir de l'échappement (11) sur une valeur pré-déterminée, caractérisée en ce que le réservoir est prévu extérieurement par rapport au soufflet (1) dans le corps de pompe (2), la chambre de réception du propulseur étant définie par l'intérieur du soufflet, et en ce que la vanne de commande de débit est une vanne marche/arrêt (6, 7), normalement asservie en position de fermeture et pouvant être mise en action par un électro-aimant extérieur (9).

2. Une micro-pompe selon la revendication 1, caractérisée en ce que l'admission de liquide (3) est disposée de façon à ne pas empiéter sur le soufflet (1) dans la direction dans laquelle un pointeau injecteur est insérable dans l'admission pour la fourniture de liquide sur celle-ci.

3. Une micro-pompe selon la revendication 1 ou 2, caractérisée en ce que des moyens de régulation de débit (12, 14, 16, 18, 19, 21, 23, 24) sont prévus dans le corps de pompe pour le contrôle du régime de débit du liquide durant l'ouverture de la vanne de commande de débit (6, 7).

4. Une micro-pompe selon la revendication 3, caractérisée en ce que le système de régulation de débit est raccordé entre le réservoir (5) et la vanne de commande de débit (6, 7), et comprend un élément de soupape (16) poussé vers un siège de soupape (18) par un moyen générateur de force élastique (14) et repoussé du siège de soupape (18) de façon à autoriser un passage du liquide par l'élément de soupape (16) en réponse à une pression exercée sur un diaphragme (24) sur le côté aval, afin de maintenir une pression du liquide fourni sur la vanne de commande de débit (6, 7) à une valeur pré-déterminée.

5. Une micro-pompe selon la revendication 4, caractérisée en ce que le siège de soupape (18) est formé dans un corps cylindrique présentant un alésage axial pour diriger le liquide vers le diaphragme (24) et une surface d'extrémité plate annulaire en regard de l'élément de soupape (16), la surface plate annulaire pouvant s'engager de manière étanche aux liquides avec une surface plate de l'élément de soupape (16) qui est parallèle à la dite surface plate annulaire.

# FIG. I

FIG.3

FIG.2

2

# FIG. 4

22  24  21  20  23

25

17 18 15 14 16 19

12

13

# FIG. 5

12

IV  IV

22

# FIG. 6

26    27    28   30

# FIG.7

VI    VI

27

28

29